# EUROPÄISCHE PATENTANMELDUNG

(11) **EP 1 354 865 A1**
(43) Veröffentlichungstag der Anmeldung: **22.10.2003**
(21) Anmeldenummer: 03008308.3
(22) Anmeldetag: 10.04.2003
(51) Int. Cl.: C07C 45/40, C07C 45/60, C07C 51/09, C07C 51/41

(54) **Verfahren zur Herstellung aromatischer Aldehyde durch Ozonolyse von aromatischen Alkenen**

(30) Priorität: 16.04.2002 DE 10216967
(71) Anmelder: Bayer Aktiengesellschaft, 51368 Leverkusen (DE)
(72) Erfinder: Antons, Stefan, Dr., 51373 Leverkusen (DE); Rehse, Joachim, Dr., 42799 Leichlingen (DE); Diehl, Herbert, Dr., 51375 Leverkusen (DE); Laue, Christian, Dr., 40789 Monheim (DE)

(57) **Zusammenfassung**

Bereitgestellt wird ein neues Verfahren zur Herstellung spezieller aromatischer Aldehyde durch Ozonolyse aromatischer Alkene. Dieses neue Verfahren lässt sich vorteilhaft in eine Synthese spezieller chiraler Diole unter Einsatz derartiger aromatischer Aldehyde integrieren.

## Beschreibung

Die Erfindung betrifft ein Verfahren zur Herstellung spezieller aromatischer Aldehyde durch Ozonolyse entsprechender aromatischer Alkene sowie die Verwendung derartiger aromatischer Aldehyde in einem Verfahren zur Herstellung spezieller chiraler Diole.

Verschiedene Verbindungen der allgemeinen Formel (VIa) 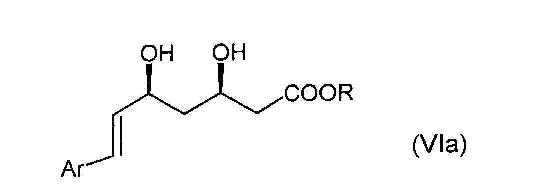 werden aufgrund ihrer Eigenschaft, die Chlosterinbiosynthese zu hemmen, in Arnzneimitteln als HMG-CoA Reduktase Inhibitoren zur Behandlung von Lipoproteinämie eingesetzt.

Bekannt sind beispielsweise

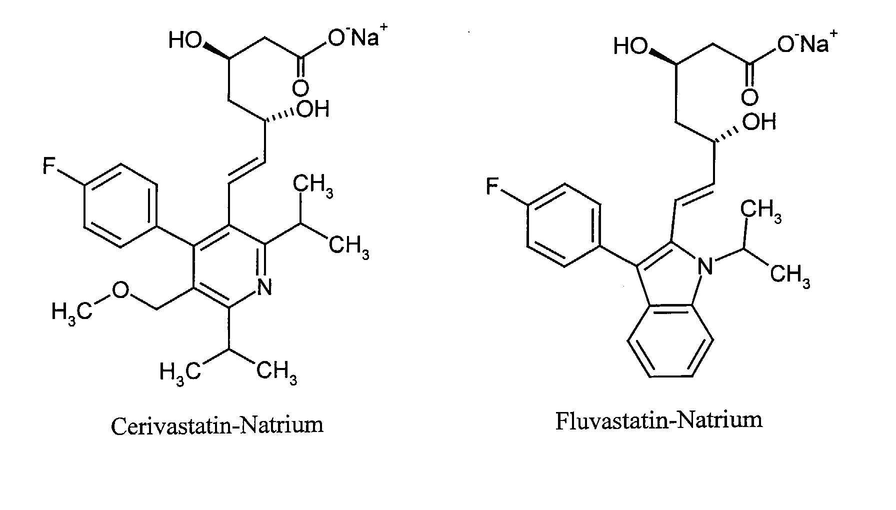

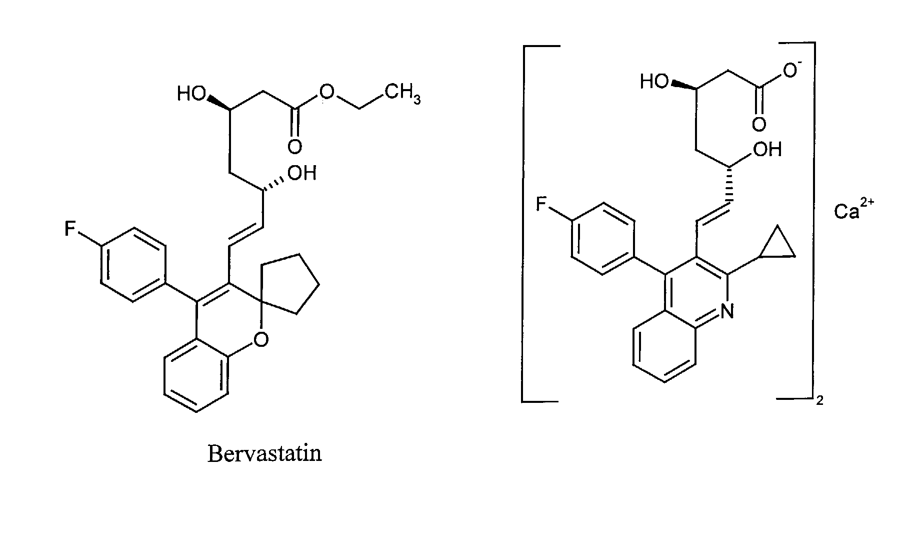

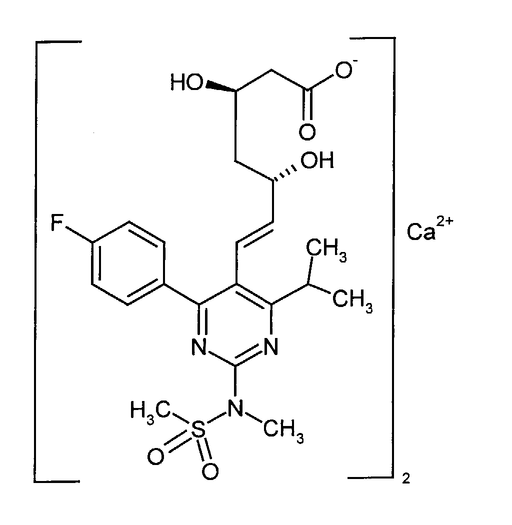

Bei diesen Verbindungen der allgemeinen Formel (VIa) wird die chirale Seitenkette üblicherweise durch folgende Synthesesequenz aufgebaut:

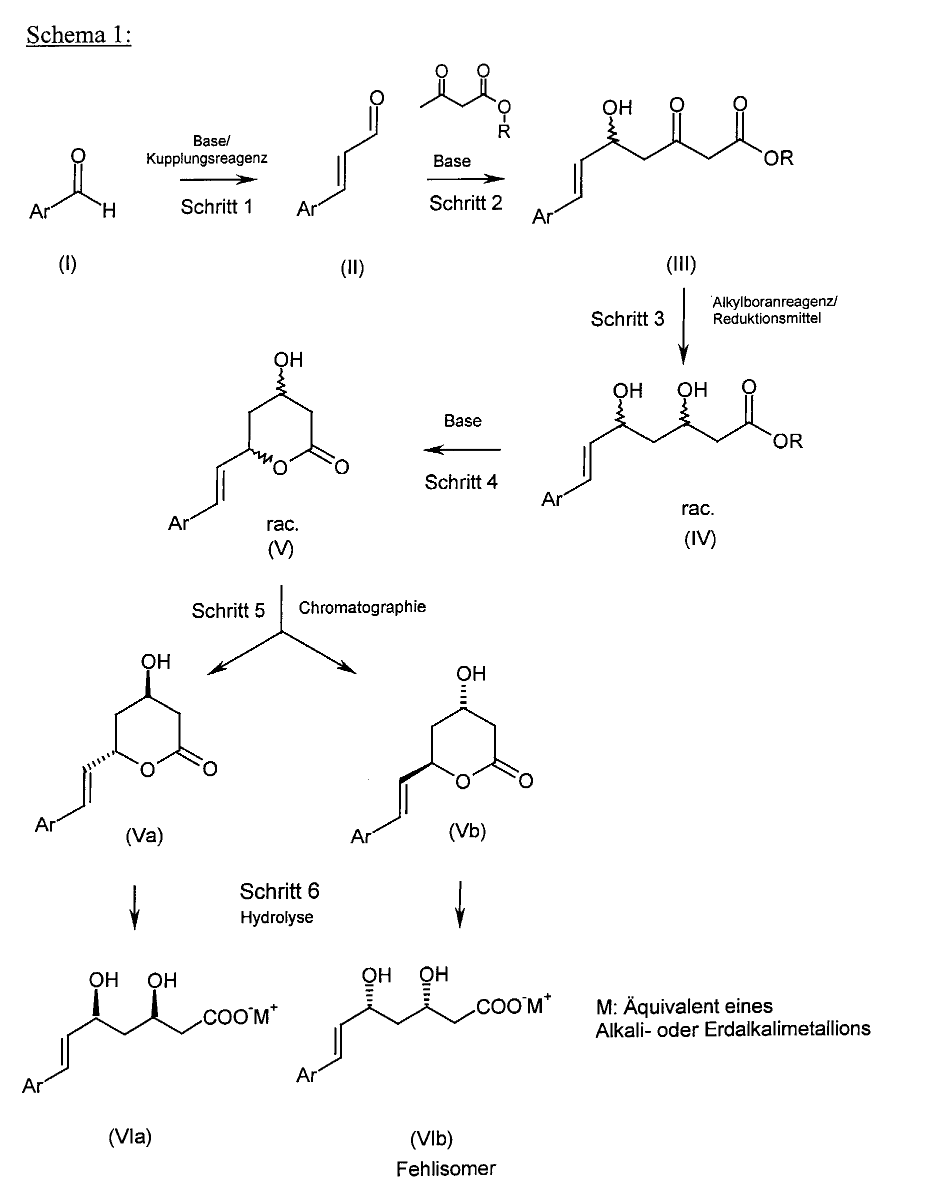

Hierbei steht Ar für einen substituierten oder unsubstituierten Aryl- oder Heteroarylrest steht.

Die Synthese der Schritte 1-3 gemäß Schema 1 ist aus EP-A-0 603 699, EP-A-0 325 130 sowie DE-A-40 40 026 bekannt.

Die Schritte 4 und 5 sind in EP-A-0 617 019 offenbart. Schritt 4 umfasst eine Überführung der racemischen Ester der allgemeinen Formel (IV) in die racemischen Lactone der allgemeinen Formel (V). In Schritt 5 wird dieses Racemat durch Chromatographie an einer chiralen Phase in die einzelnen enantiomeren Lactone separiert und die enantiomeren Lactone werden jeweils durch Hydrolyse in die enantiomerenreinen Endprodukte (VIa) und (VIb) überführt.

Durch die in Schritt 5 vorzunehmende Racemattrennung ist die Gesamtsynthese mit dem Nachteil verbunden, dass die Gesamtausbeute durch das anfallende Fehlisomer der allgemeinen Formel (VIb) erheblich reduziert wird. Hierdurch wird die wirtschaftliche Attraktivität der Gesamtsynthese deutlich reduziert, da das Fehlisomer einer Verbrennung zugeführt werden muss.

Die Aufgabe der vorliegenden Erfindung bestand darin, das Fehlisomer einer weiteren Nutzung zuzuführen.

Gegenstand der vorliegenden Erfindung ist ein Verfahren zur Herstellung aromatischer Aldehyde der Formel (I) 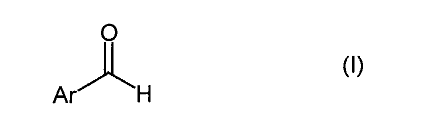 wobei
- Ar: für einen substituierten oder unsubstituierten Aryl- oder Heteroarylrest steht,
durch Umsetzung von Verbindungen der allgemeinen Formel (VIb) oder (Vb) 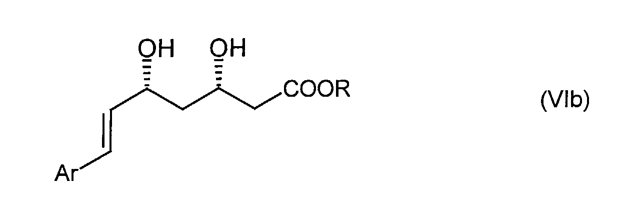 oder 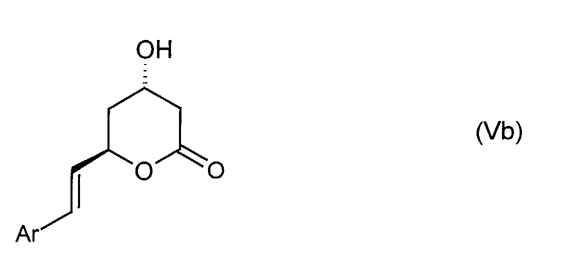 wobei
- Ar: die für allgemeine Formel (I) genannte Bedeutung hat und
- R: Wasserstoff, geradkettiges oder verzweigtes C₁-C₆-Alkyl, C₃-C₆-Cycloalkyl oder ein Äquivalent eines Alkali- oder Erdalkalimetalls bedeutet,
mit Ozon.

In den Verbindungen der allgemeinen Formeln (Vb) und (VIb) steht Aryl für einen substituierten oder unsubstituierten Aryl- oder Heteroarylrest.

Ar steht beispielsweise für einen Pyridyl-, Pyrimidyl-, Pyridazin-, Pyrazin-, Pyrrol-, Triazol-, Chinolin-, Indol-, Chromen- oder Chromanrest, der ein- oder mehrfach, gleich oder verschieden substituiert sein kann. Als Substituenten sind weitere Aryl-, Alkyl- Cycloalkyl-, Halogenalkyl-, Halogenaryl-, O-, N- und S-Alkylreste möglich.

In den Verbindungen der allgemeinen Formeln (Vb) und (VIb) steht R bevorzugt für Methyl, Ethyl, n-Propyl, iso-Propyl, n-Butyl, iso-Butyl oder tert.Butyl.

Besonders bevorzugt wird das erfindungsgemäße Verfahren durchgeführt unter Einsatz von Verbindungen der allgemeinen Formel (Vb) oder (VIb), bei denen Ar für einen Rest der Formel 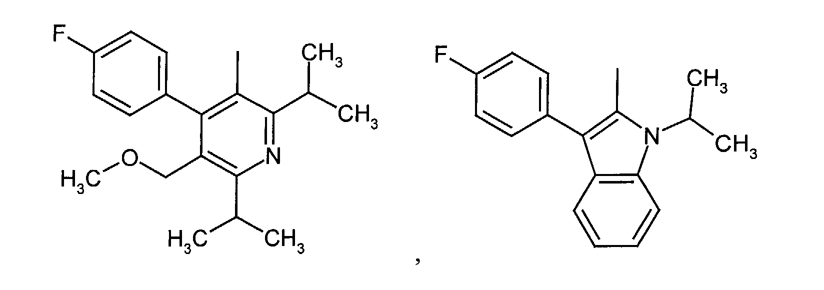 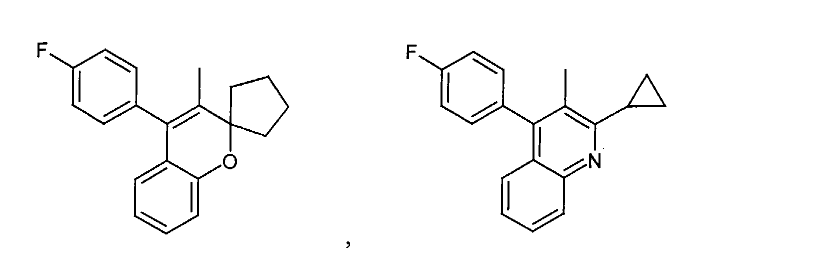 oder 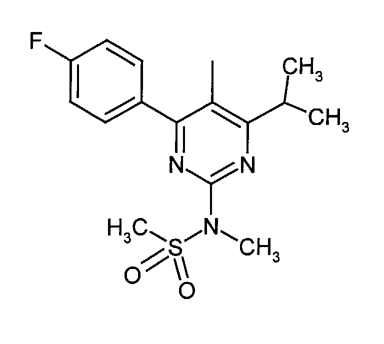 steht.

Die Ozonolyse der Verbindungen der allgemeinen Formel (Vb) oder (VIb) wird üblicherweise in einem oder mehreren inerten polaren Lösungsmitteln durchgeführt.

Geeignet sind beispielsweise Alkohole, insbesondere Methanol oder Ethanol, organische Carbonsäuren, bevorzugt organische C₁-C₆-Carbonsäuren, insbesondere Ameisensäure oder Essigsäure, Aldehyde, bevorzugt C₃-C₆-Aldehyde, insbesondere Formaldehyd oder Acetaldehyd, Ketone, bevorzugt Aceton, Methylethylketon, Methylisobutylketon oder Cyclohexanon.

Das aromatische Alken der Formel (Vb) oder (VIb) wird in dem inerten polaren Lösungsmittel vorgelegt und das Ozon in diese Lösung eingeleitet. Die Temperatur des Reaktionsgemisches liegt dabei im Bereich von - 100 bis + 30°C, bevorzugt im Bereich von - 40 bis + 20°C. Bewährt haben sich Dosiergeschwindigkeiten von 0,01 bis 10 Mol Ozon/Mol Alken/Stunde, bevorzugt von 0,1 bis 1 Mol Ozon /Mol Alken/Stunde. Üblicherweise werden Ozon und das aromatische Alken der Formel (Vb) oder (VIb) insgesamt in einem molaren Verhältnis von (1:1) - (1:10), bevorzugt (1:1) - (1:2) eingesetzt.

Nach Beendigung der Ozon-Einleitung wird das Reaktionsgemisch aufgearbeitet. Hierbei hat sich folgende Vorgehensweise bewährt: Gegebenenfalls vorhandenes überschüssiges Ozon wird mit einer Schwefelverbindung wie Dimethylsulfid, einem Alkalisulfid oder Alkalihydrogensulfid zerstört. Nach Entfernung der Lösungsmittel wird entweder durch Destillation, Chromatographie oder Kristallisation gereinigt.

Günstigerweise wird durch die erfindungsgemäße Ozonolyse der aromatische Aldehyd der Formel (I) gewonnen, welcher das Edukt für die Synthese der chiralen Seitenkette gemäß Schema 1 darstellt. Möglich ist somit eine Rückführung des durch die erfindungsgemäße Ozonolyse gewonnenen aromatischen Aldehyds in die Gesamtsynthese gemäß Schema I. Hierdurch steigt die wirtschaftliche Attraktivität des Gesamtverfahrens ganz erheblich.

Gegenstand der Erfindung ist daher auch ein Verfahren zur Herstellung von Verbindungen der allgemeinen Formel (VIa) durch
(1) Umsetzung eines Aldehyds der allgemeinen Formel (I) 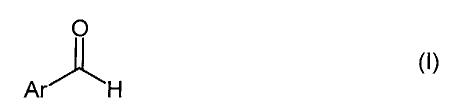 wobei
   - Ar: für einen substituierten oder unsubstituierten Aryl- oder Heteroarylrest steht,
   mit einem Phosphonatester oder Triphenylphosphoniumsalz als Kupplungsreagenz in Gegenwart einer organischen oder anorganischen Base unter Bildung einer Verbindung der allgemeinen Formel (II), 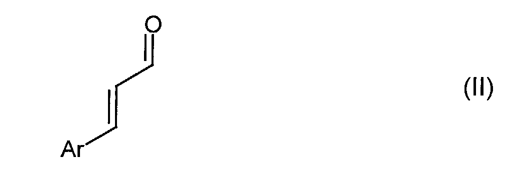 wobei Ar die für die Formel (I) genannte Bedeutung besitzt,
(2) Umsetzung der Verbindung der allgemeinen Formel (II) mit einem Acetessigester eines geradkettigen oder verzweigten C₁-C₆-Alkohols in Gegenwart einer Base unter Bildung einer Verbindung der allgemeinen Formel (III) 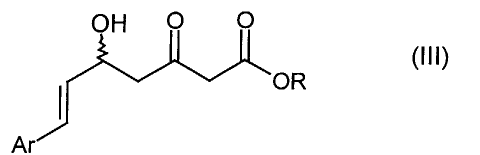 wobei
   - Ar: die für die Formel (I) genannte Bedeutung besitzt und
   - R: für einen geradkettigen oder verzweigten C₁-C₆-Alkylrest steht,
(3) Umsetzung der Verbindung der allgemeinen Formel (III) mit einem Alkylboranreagenz in Gegenwart eines Reduktionsmittels unter Bildung eines Racemates der allgemeinen Formel (IV) 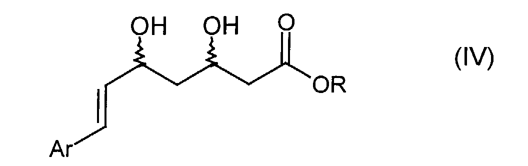 wobei
   - Ar: die für die Formel (I) genannte Bedeutung besitzt und
   - R: die für die Formel (III) genannte Bedeutung besitzt,
(4) Umsetzung des Racemates der allgemeinen Formel (IV) mit einer Base und Cyclisierung unter Bildung eines Racemates der allgemeinen Formel (V) 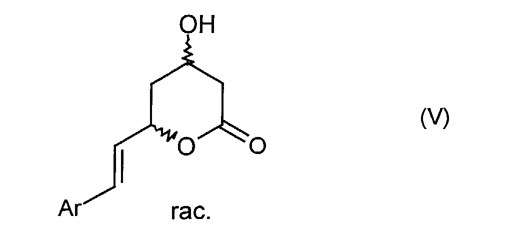 wobei
   - Ar: die für die Formel (I) genannte Bedeutung besitzt,
(5) chromatographische Auftrennung des Racemates der allgemeinen Formel (V) in die enantiomeren Lactone der allgemeinen Formeln (Va) und (Vb) 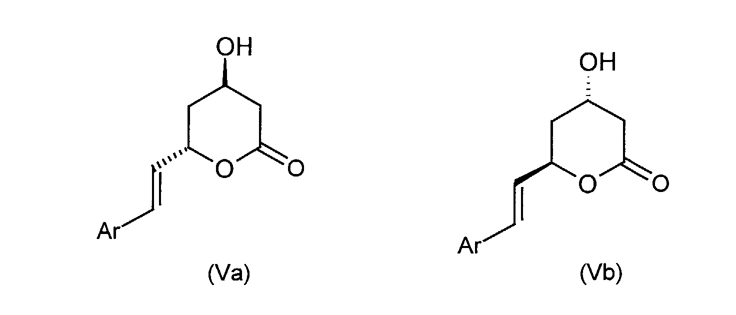 wobei
   - Ar: die für die Formel (I) genannte Bedeutung besitzt,
(6) separate Hydrolyse dieser enantiomeren Lactone der allgemeinen Formeln (Va) und (Vb) unter Bildung der Verbindungen der allgemeinen Formeln (VIa) und (VIb) 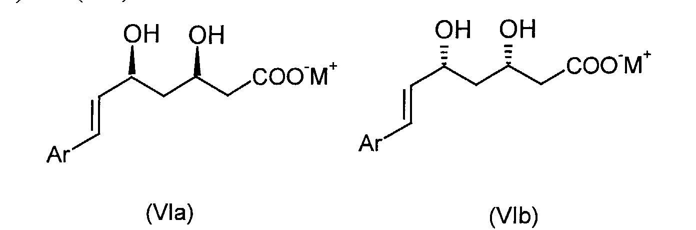 wobei
   - Ar: die für die Formel (I) genannte Bedeutung besitzt, und
   - M: für das Äquivalent eines Alkali- oder Erdalkalimetalls steht.
(7) Umsetzung einer Verbindung der allgemeinen Formel (Vb) oder (VIb) 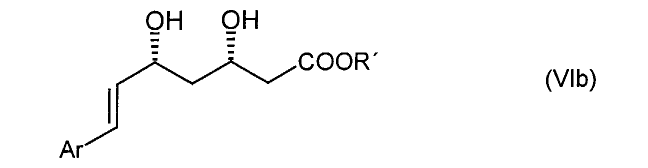 oder 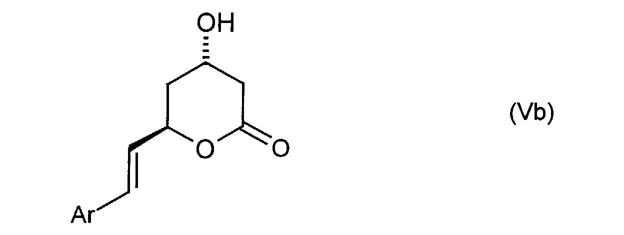 wobei
   - Ar: die für die Formel (I) genannte Bedeutung besitzt und
   - R': Wasserstoff, geradkettiges oder verzweigtes C₁-C₆-Alkyl, C₃-C₆-Cycloalkyl oder ein Äquivalent eines Alkali- oder Erdalkalimetalls bedeutet,
   mit Ozon unter Bildung des Aldehyds der allgemeinen Formel (I) und
(8) Rückführung dieses Aldehyds der allgemeinen Formel (I) in Schritt (1).

In **Schritt 1** wird zunächst der Aldehyd mit einem Phosphonatester oder Triphenylphosphoniumsalz als Kupplungsreagenz in Gegenwart einer organischen oder anorganischen Base umgesetzt. Besonders bevorzugt sind Phosphonatester als Kupplungsreagenz.

Als anorganische Basen können Alkalihydride, -hydroxide, -carbonate oder -hydrogencarbonate eingesetzt werden. Als organische Basen haben sich Trialkylamine oder Pyridinderivate bewährt.

Die Behandlung des Aldehyds erfolgt im allgemeinen in einem Temperaturbereich von - 100 bis + 100°C, bevorzugt von -20 bis + 100°C, besonders bevorzugt von 0 bis + 50°C.

Als Lösungsmittel bei der Behandlung mit der Base eignen sich Ether wie beispielsweise Diethylether, Dioxan oder Tetrahydrofuran oder auch Mischungen derselben.

Das gebildete Acrolein der allgemeinen Formel (II) wird aus der Reaktionsmischung durch Zusatz einer Säure, bevorzugt einer anorganischen Säure freigesetzt. Eine Reinigung erfolgt bevorzugt durch Kristallisation oder Destillation.

**In Schritt 2** wird das erhaltene Acrolein der allgemeinen Formel (II) mit einem Acetessigester eines geradkettigen oder verzweigten C₁-C₆-Alkohols in Gegenwart einer Base, bevorzugt einer starken Base, umgesetzt. Besonders bevorzugt sind NaH und Butyllithium. Alternativ kann auch das Natriumsalz eines Acetessigesters eingesetzt werden.

Die Behandlung des Acroleins der allgemeinen Formel (II) erfolgt im allgemeinen in einem Temperaturbereich von - 100 bis + 100°C, bevorzugt von -50 bis +20°C, besonders bevorzugt von -30 bis + 20°C.

Als Lösungsmittel bei der Behandlung mit der Base eignen sich Ether wie beispielsweise Diethylether, Dioxan oder Tetrahydrofuran. Besonders bevorzugt wird Tetrahydrofuran eingesetzt.

Während dieser Umsetzung wird eine Verbindung der allgemeinen Formel (III) gebildet, bei der es sich um einen racemischen Hydroxyketoester handelt. Die Freisetzung der Verbindung der allgemeinen Formel (III) aus der Reaktionsmischung erfolgt durch Zusatz einer Säure, bevorzugt einer anorganischen Säure. Eine Reinigung erfolgt bevorzugt durch Kristallisation oder Destillation.

In **Schritt 3** wird der in Schritt 2 erhaltene Hydroxyketoester der allgemeinen Formel (III) mit einem Alkylboranreagenz in Gegenwart eines Reduktionsmittels zu einer Verbindung der allgemeinen Formel (IV) umgesetzt, bei der es sich um einen racemischen Dihydroxyester handelt.

Als Reduktionsmittel eignen sich komplexe Metallhydride, wie beispielsweise Lithiumaluminiumhydrid, Natriumcyanoborhydrid, Natriumaluminiumhydrid, Diisobutylaluminiumhydrid, Natriumboranat oder Natrium-bis-(2-methoxy-ethoxy)-dihydroaluminat.

Die Behandlung des Hydroxyketoesters in Schritt 3 erfolgt im allgemeinen in einem Temperaturbereich von -100 bis 0°C, bevorzugt von -80 bis -21°C und besonders bevorzugt von -78 bis -50°C.

Als Lösungsmittel eignen sich Ether wie beispielsweise Diethylether, Dioxan oder Tetrahydrofuran. Besonders bevorzugt wird Tetrahydrofuran eingesetzt.

Der gebildete racemische Dihydroxyester der allgemeinen Formel (IV) wird durch Zusatz einer Säure, bevorzugt einer anorganischen Säure freigesetzt. Eine Reinigung erfolgt bevorzugt durch Kristallisation oder Destillation.

In **Schritt 4** werden die racemischen Dihydroxyester der allgemeinen Formel (IV) in die racemischen Lactone der Formel (V) überführt.

Diese Überführung erfolgt im allgemeinen durch Behandeln des Esters mit Basen und anschließende Cyclisierung in geeigneten Lösungsmitteln unter Wasserabspaltung. Bei dieser Reaktion entstehen intermediär die Carbonsäuren und deren Salze.

Als Basen hierfür eignen sich die üblichen anorganischen Basen wie Natriumhydroxid, Kaliumhydroxid, Natriumcarbonat oder Kaliumcarbonat. Bevorzugt wird Natriumhydroxid eingesetzt.

Als Lösungsmittel bei der Behandlung mit der Base eignen sich Ether wie beispielsweise Diethylether, Dioxan oder Tetrahydrofuran. Besonders bevorzugt wird Tetrahydrofuran eingesetzt.

Die anschließende Cyclisierung durch Erhitzen erfolgt im allgemeinen in Lösungsmitteln, die sich unter den Reaktionsbedingungen inert verhalten. Hierzu gehören Kohlenwasserstoffe wie Benzol, Toluol, Xylol, Erdölfraktionen, Tetralin, Diglym oder Triglym. Besonders bevorzugt werden Benzol, Toluol und Xylol eingesetzt. Ebenso ist es möglich, Mischungen der genannten Lösungsmittel einzusetzen. Besonders bevorzugt verwendet man Toluol. Das hierbei abgespaltene Wasser kann durch azeotrope Destillation oder mittels Molsieb entfernt werden. Besonders bevorzugt wird die azeotrope Destillation.

Die Behandlung der Ester der allgemeinen Formel (IV) mit Basen erfolgt im allgemeinen in einem Temperaturbereich von 0-50°C, bevorzugt von 10-30°C, besonders bevorzugt bei 20°C.

Die Cyclisierung erfolgt im allgemeinen in einem Temperaturbereich von 0 bis + 200°C, bevorzugt von + 25 bis + 150°C. Bevorzugt wird bei der Siedetemperatur des azeotropen Gemisches des jeweils verwendeten Lösungsmittels mit Wasser gearbeitet.

In **Schritt 5** werden dann die Enantiomere des Racemates (V) durch Chromatographie an einer chiralen Phase in die einzelnen enantiomeren Lactone separiert und die enantiomeren Lactone anschließend durch Hydrolyse in die enantiomerenreinen Endprodukte bzw. deren Alkali- oder Erdalkalisalze überführt.

Die chromatographische Trennung der racemischen Latone in die einzelnen enantiomerenreinen Lactone erfolgt im allgemeinen an üblichen chiralen Materialien. Hierzu gehören bevorzugt optisch aktive Polymere von optisch aktiven (Meth)-acrylsäure Derivaten. Besonders bevorzugt sind hier Polymerisate von optisch aktiven N-(Meth)acryloyl-aminosäure Derivaten, wie sie in EP-A-0 379 917 beschrieben sind. Ganz besonders bevorzugt seien hier Polymerisate aus folgenden optisch aktiven N-Acryloyl-aminosäureestem genannt: N-Acryloyl-L- bzw. D-aminosäurementhylester, wobei als Aminosäure z.B. Leucin, Alanin, Phenylalanin, Valin oder sonstige Aminosäuren in Frage kommen.

Als Fließmittel für die Trennung des Racemats werden übliche organische Lösungsmittel bzw. Lösungsmittelgemische verwendet, die das als Adsorbens eingesetzte Polymerisat anquellen und das zu benutzende Racemat lösen. Beispielsweise seien genannt: Kohlenwasserstoffe wie Benzol, Toluol oder Xylol, Ether wie Diethylether, Dioxan oder Tetrahydrofuran, Halogenkohlenwasserstoffe wie Di- oder Trichlormethan, Aceton, Acetonitril oder Essigester oder aber Gemische der genannten Lösungsmittel. Als besonders geeignet haben sich Mischungen aus Toluol und Tetrahydrofuran sowie aus Toluol und Dioxan erwiesen.

Die Hydrolyse des jeweiligen enantiomerenreinen Lactons in das gewünschte enantiomerenreine Endprodukt gemäß Schritt 6 erfolgt in üblicher Weise unter Verwendung einer Base in organischen Lösungsmitteln.

Als Lösungsmittel kommen hierbei die üblichen organischen Lösungsmittel in Betracht, die sich unter den Reaktionsbedingungen inert verhalten. Bevorzugt seien hier Ether wie Diethylether, Dioxan oder Tetrahydrofuran genannt. Besonders bevorzugt wird Tetrahydrofuran eingesetzt.

Als Basen eignen sich die üblichen anorganischen Basen wie Alkalihydroxide oder Alkalicarbonate. Bevorzugt sind Natriumhydroxid oder Kaliumhydroxid.

Die Hydrolyse erfolgt im allgemeinen in einem Temperaturbereich von 0 - 60°C, bevorzugt von 10 bis 50°C, besonders bevorzugt bei 20°C.

Nach Durchführung der Schritte 2-4 des erfindungsgemäßen Verfahrens werden jeweils Reaktionsgemische erhalten, die neben den jeweiligen Produkten der Einzelschrittes, d.h. den Verbindungen der allgemeinen Formeln (III), (IV) und (V), auch noch nicht umgesetzte Edukte, d.h. Verbindungen der allgemeinen Formeln (II), (III) und/oder (IV) enthalten. Das nach Abtrennung der gewünschten Produkte verbleibende Reaktionsgemisch ("Mutterlauge") kann ebenfalls einer Ozonolyse unterzogen werden, wobei ebenfalls der Aldehyd der allgemeinen Formel (I) erhalten wird.

Gegenstand der vorliegenden Erfindung ist somit auch ein Verfahren zur Herstellung aromatischer Aldehyde der Formel (I) 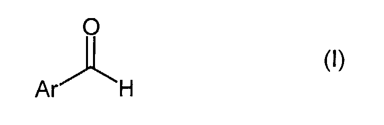 wobei
- Ar: für einen substituierten oder unsubstituierten Aryl- oder Heteroarylrest steht,
durch Umsetzung eines Reaktionsgemisches enthaltend eine oder mehrere der Verbindungen der allgemeinen Formel (VII) 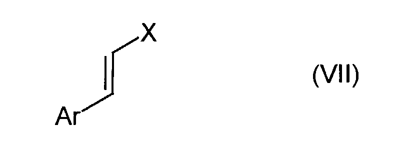 wobei
- - X: für 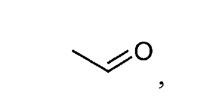 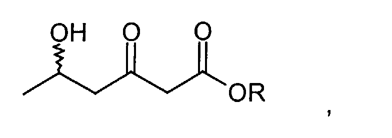 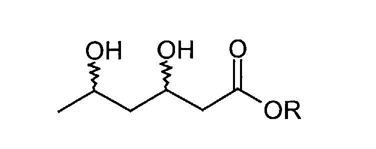 oder 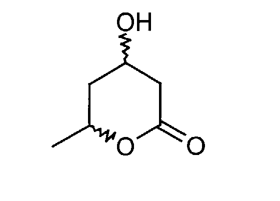 steht,
mit Ozon.

Die Bedingungen einer derartigen Ozonolyse der Mutterlaugen können analog zu den genannten Bedingungen der Ozonolyse der Verbindungen der Formeln (Vb) oder (VIb) gewählt werden. Diese zusätzliche Ozonolyse von einer oder mehrerer der Mutterlaugen aus den Schritten 2 bis 4 des erfindungsgemäßen Verfahrens kann einzeln oder in vereinigter Form, anstelle oder aber zusätzlich zur Ozonolyse der Verbindungen der allgemeinen Formeln (Vb) oder (VIb) durchgeführt werden. Hierdurch wird die Gesamtausbeute des Verfahrens weiter erhöht.

Gegenstand der Erfindung ist somit auch ein Verfahren zur Herstellung von Verbindungen der allgemeinen Formel (VIa) durch
(1) Umsetzung eines Aldehyds der allgemeinen Formel (I) 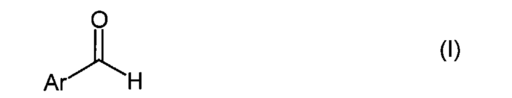 wobei
   - Ar: für einen substituierten oder unsubstituierten Aryl- oder Heteroarylrest steht,
   mit einem Phosphonatester oder Triphenylphosphoniumsalz als Kupplungsreagenz in Gegenwart einer organischen oder anorganischen Base unter Bildung einer Verbindung der allgemeinen Formel (II), 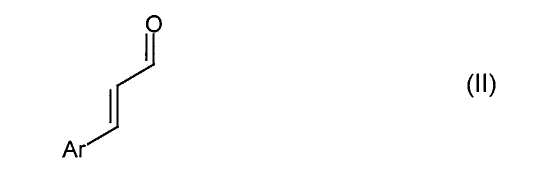 wobei Ar die für die Formel (I) genannte Bedeutung besitzt,
(2) Umsetzung der Verbindung der allgemeinen Formel (II) mit einem Acetessigester eines geradkettigen oder verzweigten C₁-C₆-Alkohols in Gegenwart einer Base unter Bildung einer Verbindung der allgemeinen Formel (III) 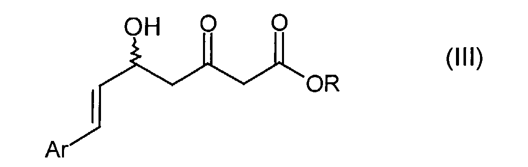 wobei
   - Ar: die für die Formel (I) genannte Bedeutung besitzt und
   - R: für einen geradkettigen oder verzweigten C₁-C₆-Alkylrest steht,
(3) Umsetzung der Verbindung der allgemeinen Formel (III) mit einem Alkylboranreagenz in Gegenwart eines Reduktionsmittels unter Bildung eines Racemates der allgemeinen Formel (IV) 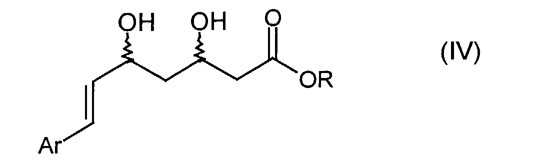 wobei
   - Ar: die für die Formel (I) genannte Bedeutung besitzt und
   - R: die für die Formel (III) genannte Bedeutung besitzt,
(4) Umsetzung des Racemates der allgemeinen Formel (IV) mit einer Base und Cyclisierung unter Bildung eines Racemates der allgemeinen Formel (V) 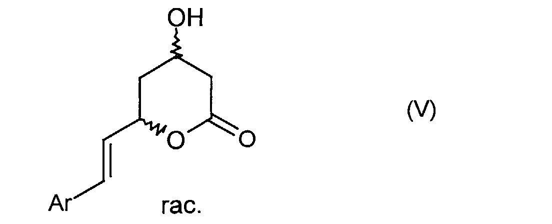 wobei
   - Ar: die für die Formel (I) genannte Bedeutung besitzt,
(5) chromatographische Auftrennung des Racemates der allgemeinen Formel (V) in die enantiomeren Lactone der allgemeinen Formeln (Va) und (Vb) 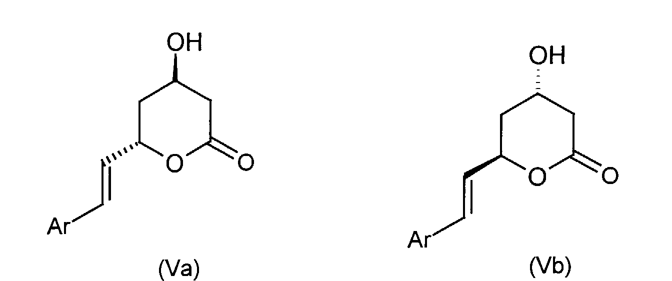 wobei
   - Ar: die für die Formel (I) genannte Bedeutung besitzt,
(6) separate Hydrolyse dieser enantiomeren Lactone der allgemeinen Formeln (Va) und (Vb) unter Bildung der Verbindungen der allgemeinen Formel (VIa) und (VIb) 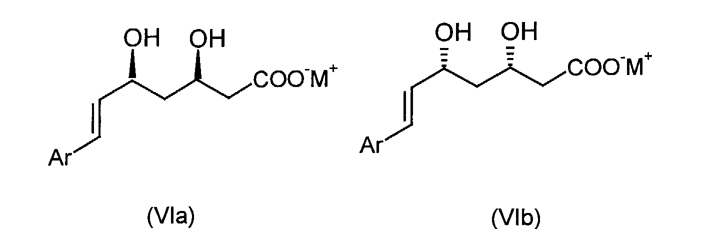 wobei
   - Ar: die für die Formel (I) genannte Bedeutung besitzt, und
   - M: für das Äquivalent eines Alkali- oder Erdalkalimetalls steht.
(7) Umsetzung eines oder mehrerer der Reaktionsgemische, die nach den Schritten (2), (3) und/oder (4) nach Abtrennung der Verbindungen der Formeln (III), (IV) bzw. (V) erhalten werden, enthaltend eine oder mehrere der Verbindungen der allgemeinen Formel (VII) 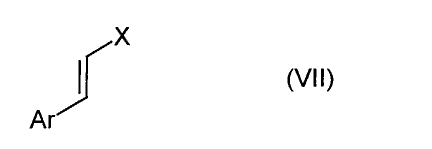 wobei
   - - X: für 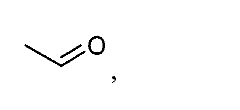 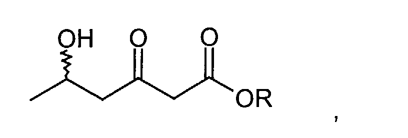 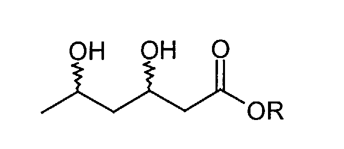 oder 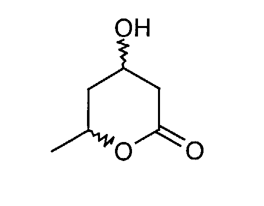 steht,
   einzeln oder in vereinigter Form mit Ozon unter Bildung des Aldehyds der allgemeinen Formel (I) und
(8) Rückführung dieses Aldehyds der allgemeinen Formel (I) in Schritt (1).

### Beispiele

### Beispiel 1

### Ozonolyse von (E)-6-{2-(2,6-Diisopropyl-4-(4-fluorphenyl)-3-methoxymethyl-pyrid-5-yl)-ethenyl)-3,4,5,6-tetrahydro-4-hydroxy-2H-pyran-2-on (1) zu 2,6-Diisopropyl-4-(4-fluorphenyl)-5-methoxymethyl-pyridin-3-carbaldehyd (2)

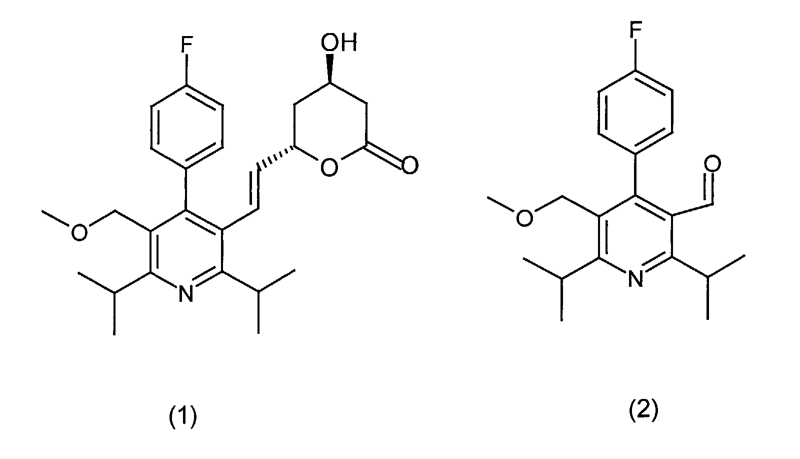

In eine auf -40°C gekühlte Lösung von 4,0 g (9,1 mmol) des Alkens (1) in 160 ml Methanol p.a. leitet man unter Rühren Ozon ein. Anwendung findet hierbei ein Ozon-Generator der Fa. Fischer. Die Ozondosierungsgeschwindigkeit liegt bei 30 1 O₂/h und ca. 2 g O₃/h. Nach 17 min (entsprechend 11 mmol O₃) wird die Ozonolyse abgebrochen, als eine leichte Blaufärbung der Reaktionsmischung eingetreten ist. Das Reaktionsgemisch wird bei - 40°C mit Stickstoff überspült und bei dieser Temperatur mit 600 mg (9,67 mmol) Dimethylsulfid versetzt. Man lässt die Reaktionsmischung innerhalb von 30 min auf 20°C erwärmen und lässt die Mischung noch 2,5 h stehen. Anschließend wird das Lösungsmittel durch Destillation entfernt und der Rückstand in wenig Methylenchlorid gelöst, mit 10 g Kieselgel versetzt und vom Methylenchlorid befreit. Das mit dem gewünschten Produkt beladene Kieselgel wird auf eine Fritte mit 100 g Kieselgel geschüttet und mit 12 50 ml-Fraktionen Cyclohexan-Essigester (Volumenverhältnis 20:1) eluiert.

Die Produktfraktionen enthalten 2,5 g (83 %) eines farblosen Oels, das in Substanz kristallisiert.

### Beispiel 2

### Ozonolyse von (E)-6-{2-(2,6-Diisopropyl-4-phenyl-3-methoxymethyl-pyrid-5-yl)-ethenyl)-3,4,5,6-tetrahydro-4-hydroxy-2H-pyran-2-on (3) zu 2,6-Diisopropyl-4-phenyl-5-methoxymethyl-pyridin-3-carbaldehyd (4)

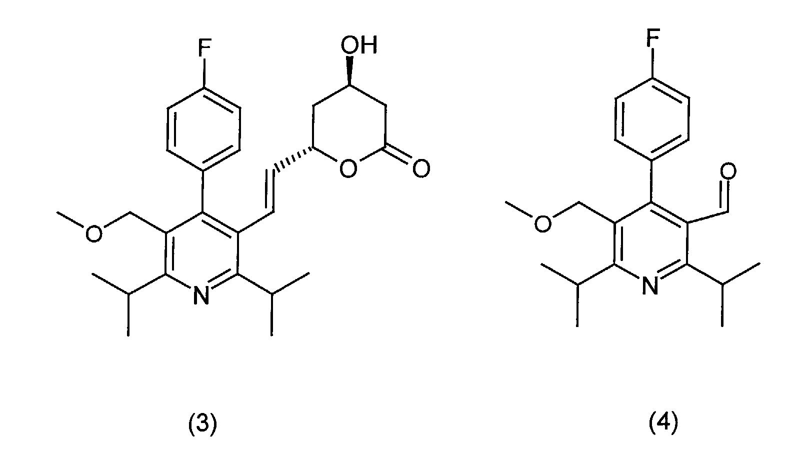

Aus dem Gesamtverfahren gemäß Schema 1 wird die Mutterlauge der Chromatographie in Schritt 5, welche zu 90 % aus dem Fehlisomeren (Vb) besteht am Rotationsverdampfer bei einem verminderten Druck von ca. 20 mbar bei einer Badtemperatur von ca. 40°C soweit wie möglich vom Lösungsmittel befreit.

Der verbleibende, dunkelbraun-ölige Rückstand wird in Isopropanol gelöst (10 g in 75 ml) und bei 20°C mit 75 ml 50 %iger Essigsäure versetzt. Die Lösung wird auf -20 bis -30°C abgekühlt. Anschließend leitet man unter Rühren für eine Dauer von 1 h Ozon ein. Anwendung findet ein Ozon-Generator der Fa. Sander. Eingeleitet wird das Ozon mit einer Dosiergeschwindigkeit von ca. 35 1 O₂/h und ca. 2 g O₃/h. Anschließend leitet man ohne Ozongenerator eine weitere Stunde O₂ durch die Reaktionslösung.

Man versetzt bei - 30°C mit ca. 10 ml Dimethylsulfid und lässt die Reaktionsmischung über 12 Stunden unter Rühren auf 20°C erwärmen. Ist der Jodid/Stärke Test auf Ozonide negativ, wird die Reaktionsmischung unter vermindertem Druck vom Lösungsmittel befreit. Ist der Jodid/Stärke-Test positiv, wird erneut mit Dimethylsulfid versetzt, bis der Test negativ ist.

Der nach dem Einengen verbleibende Rückstand wird in 250 ml Ethylacetat gelöst, mit 100 ml gesättigter NaHCO₃-Lösung, 100 ml gesättigter NaCl-Lösung und 100 ml Wasser gewaschen und anschließend über Na₂SO₄ getrocknet. Der nach der Entfernung des Lösungsmittels verbleibende Rückstand wird aus Isopropanol/H₂O 1:1 umkristallisiert.
- Ausbeute:: ca. 5 g des Aldehyds (4) entsprechend 90 % der Theorie
- Reinheit:: ca. 97 % (HPLC)

### Beispiel 3:

### Ozonolyse von Mutterlaugen aus der Umsetzung des Aldehyds (4) gemäß Schema 1

Die bei einer Umsetzung von Aldehyd (4) gemäß Schema 1 nach den Schritten 1, 2 und 3 anfallenden Mutterlaugen werden vereinigt und bei einem verminderten Druck von ca. 20 mbar bei einer Badtemperatur von ca. 40°C soweit wie möglich vom Lösungsmittel befreit. Der verbleibende, dunkelbraun-ölige Rückstand wird in Isopropanol gelöst (10 g in 75 ml) und bei 20°C mit 75 ml 50 %iger Essigsäure versetzt. Die Lösung wird auf - 20 bis - 30°C abgekühlt.

Anschließend leitet man unter Rühren ca. 1 h Ozon ein. Anwendung findet ein Ozon-Generator der Fa. Sander. Das Ozon wird mit einer Dosiergeschwindigkeit von ca. 35 l O₂/h, und ca. 2 g O₃/h eingeleitet. Anschließend leitet man ohne Ozon-Generator eine weitere Stunde O₂ durch die Reaktionslösung. Man versetzt bei - 30°C mit ca. 10 ml Dimethylsulfid und lässt die Reaktionsmischung über 12 Stunden unter Rühren auf 20°C erwärmen.

Ist der Jodid/Stärke-Test auf Ozonide negativ, wird unter vermindertem Druck das Lösungsmittel weitgehend entfernt. Ist der Jodid/Stärke-Test positiv, wird erneut mit Dimethylsulfid versetzt, bis der Test negativ ist.

Der nach weitgehender Entfernung des Lösungsmittels verbleibende Rückstand wird in 250 ml Ethylacetat gelöst, mit 100 ml gesättigter NaHCO₃-Lösung, 100 ml gesättigter NaCl-Lösung und 100 ml Wasser gewaschen und anschließend über Na₂SO₄ getrocknet. Der nach der Entfernung des Lösungsmittels verbleibende Rückstand wird aus Isopropanol/H₂O 1:1 umkristallisiert.
- Ausbeute :: ca. 5 g des Aldehyds (4) entsprechend 70 % der Theorie
- Reinheit:: ca. 97 % (HPLC)

## Patentansprüche

1. Verfahren zur Herstellung aromatischer Aldehyde der Formel (I) 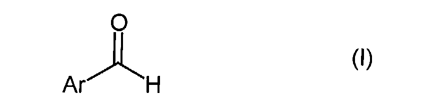 wobei
Ar für einen substituierten oder unsubstituierten Aryl- oder Heteroarylrest steht,
durch Umsetzung von Verbindungen der allgemeinen Formel (VIb) oder (Vb) 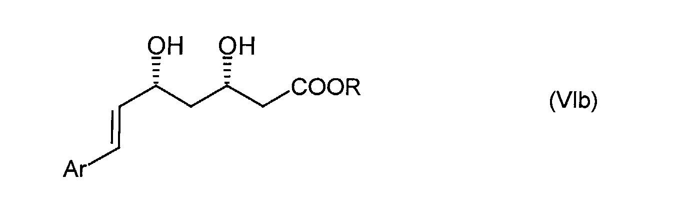 oder 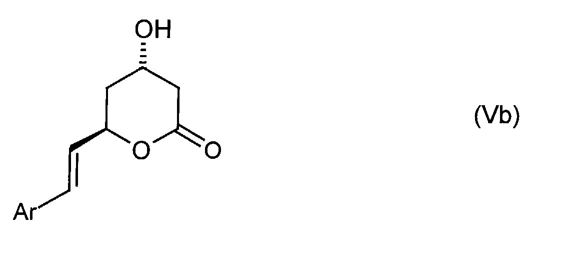 wobei
Ar die für die allgemeine Formel (I) genannte Bedeutung besitzt und
R Wasserstoff, geradkettiges oder verzweigtes C₁-C₆-Alkyl, C₃-C₆-Cycloalkyl oder ein Äquivalent eines Alkali- oder Erdalkalimetalls bedeutet,

2. Verfahren nach Anspruch 1, **dadurch gekennzeichnet, dass** Verbindungen der allgemeinen Formeln (Vb) oder (VIb) eingesetzt werden, bei denen R für Methyl, Ethyl, n-Propyl, iso-Propyl, n-Butyl, iso-Butyl oder tert.Butyl steht.

3. Verfahren nach Anspruch 1 oder 2, **dadurch gekennzeichnet, dass** Verbindungen der allgemeinen Formel (VIb) oder (Vb) eingesetzt werden, bei denen Ar für 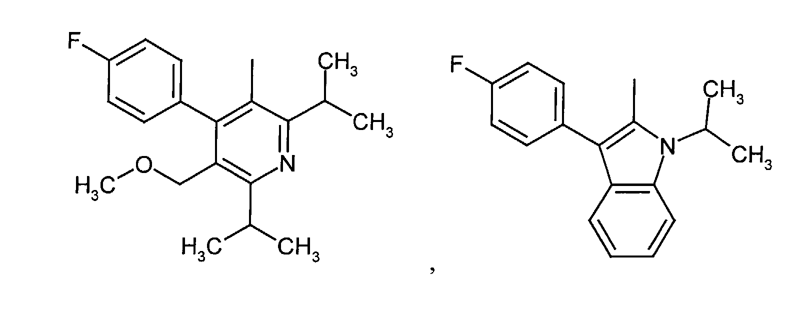 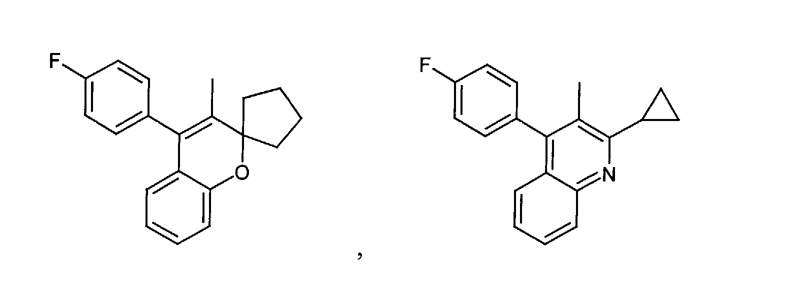 oder 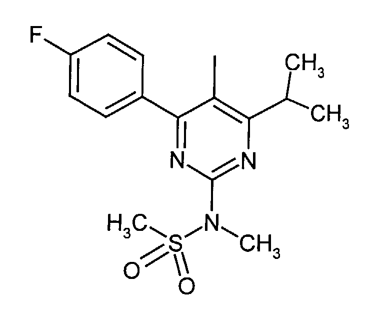 steht.

4. Verfahren nach einem oder mehreren der Ansprüche 1-3, **dadurch gekennzeichnet, dass** die Umsetzung mit Ozon in einem oder mehreren inerten polaren Lösungsmitteln durchgeführt wird.

5. Verfahren nach einem oder mehreren der Ansprüche 1-4, **dadurch gekennzeichnet, dass** die Umsetzung mit Ozon bei einer Temperatur des Reaktionsgemisches im Bereich von - 100 bis + 30°C und bevorzugt im Bereich von -40 bis + 20°C durchgeführt wird.

6. Verfahren nach einem oder mehreren der Ansprüche 1-5, **dadurch gekennzeichnet, dass** die Umsetzung mit Ozon mit einer Dosiergeschwindigkeit von 0,01 - 10 Mol Ozon/Mol Alken/Stunde und bevorzugt von 0,1 bis 1 Mol Ozon /Mol Alken/Stunde durchgeführt wird.

7. Verfahren nach einem oder mehreren der Ansprüche 1-6, **dadurch gekennzeichnet, dass** Ozon und das aromatische Alken der allgemeinen Formel (Vb) oder (VIb) insgesamt in einem molaren Verhältnis von (1:1) - (1:10), bevorzugt (1:1) - (1:2) eingesetzt werden.

8. Verfahren zur Herstellung von Verbindungen der allgemeinen Formel (VIa) 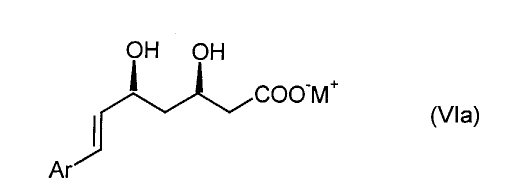 wobei
Ar für einen substituierten oder unsubstituierten Aryl- oder Heteroarylrest steht, und
M für ein Äquivalent eines Alkali- oder Erdalkalimetalls steht,
durch
(1) Umsetzung eines Aldehyds der allgemeinen Formel (I) 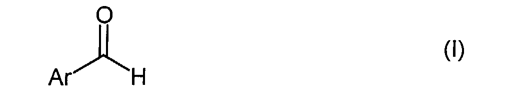 wobei
Ar für einen substituierten oder unsubstituierten Aryl- oder Heteroarylrest steht,
mit einem Phosphonatester oder Triphenylphosphoniumsalz als Kupplungsreagenz in Gegenwart einer organischen oder anorganischen Base unter Bildung einer Verbindung der allgemeinen Formel (II), 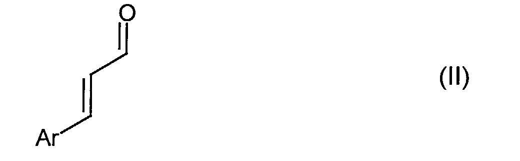 wobei Ar die für die Formel (I) genannte Bedeutung besitzt,
(2) Umsetzung der Verbindung der allgemeinen Formel (II) mit einem Acetessigester eines geradkettigen oder verzweigten C₁-C₆-Alkohols in Gegenwart einer Base unter Bildung einer Verbindung der allgemeinen Formel (III) 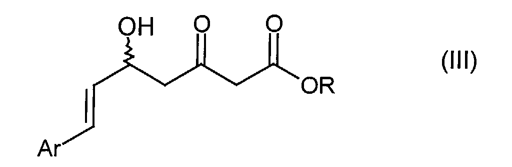 wobei
Ar die für die Formel (I) genannte Bedeutung besitzt und
R für einen geradkettigen oder verzweigten C₁-C₆-Alkylrest steht,
(3) Umsetzung der Verbindung der allgemeinen Formel (III) mit einem Alkylboranreagenz in Gegenwart eines Reduktionsmittels unter Bildung eines Racemates der allgemeinen Formel (IV) 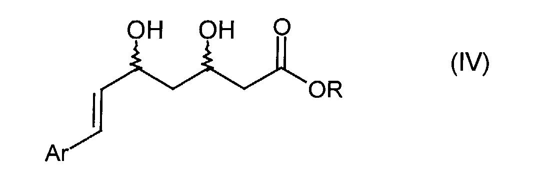 wobei
Ar die für die Formel (I) genannte Bedeutung besitzt und
R die für die Formel (III) genannte Bedeutung besitzt,
(4) Umsetzung des Racemates der allgemeinen Formel (IV) mit einer Base und Cyclisierung unter Bildung eines Racemates der allgemeinen Formel (V) 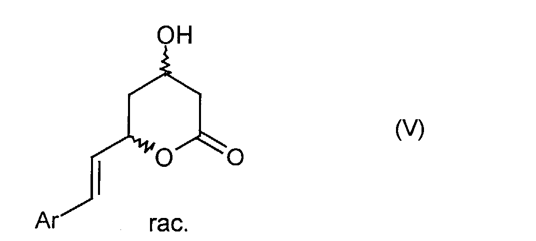 wobei
Ar die für die Formel (I) genannte Bedeutung besitzt,
(5) chromatographische Auftrennung des Racemates der allgemeinen Formel (V) in die enantiomeren Lactone der allgemeinen Formeln (Va) und (Vb) 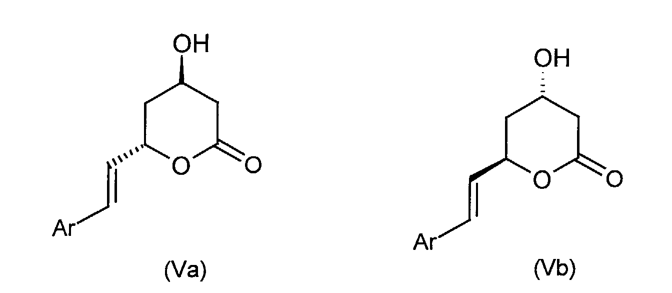 wobei
Ar die für die Formel (I) genannte Bedeutung besitzt,
(6) separate Hydrolyse dieser enantiomeren Lactone der allgemeinen Formeln (Va) und (Vb) unter Bildung der Verbindungen der allgemeinen Formel (VIa) und (VIb) 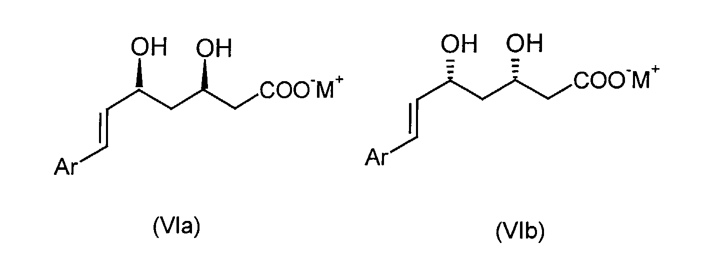 wobei
Ar die für die Formel (I) genannte Bedeutung besitzt, und
M für das Äquivalent eines Alkali- oder Erdalkalimetalls steht
(7) Umsetzung einer Verbindung der allgemeinen Formel (Vb) oder (VIb) 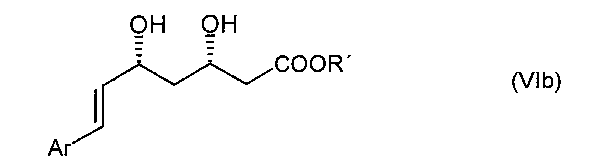 oder 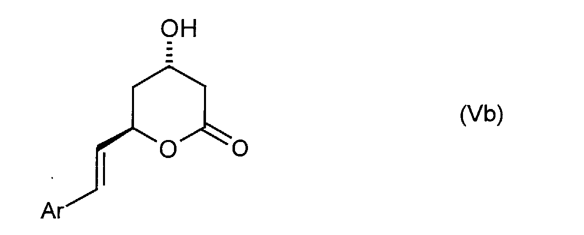 wobei
Ar die für die Formel (I) genannten Bedeutungen besitzt und
R' Wasserstoff, geradkettiges oder verzweigtes C₁-C₆-Alkyl, C₃-C₆-Cycloalkyl oder ein Äquivalent eines Alkali- oder Erdalkalimetalls bedeutet,
mit Ozon unter Bildung des Aldehyds der allgemeinen Formel (I) und
(8) Rückführung dieses Aldehyds der allgemeinen Formel (I) in Schritt (1).

9. Verfahren zur Herstellung aromatischer Aldehyde der Formel (I) 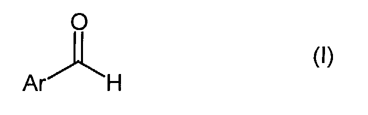 wobei
Ar für einen substituierten oder unsubstituierten Aryl- oder Heteroarylrest steht,
durch Umsetzung eines Reaktionsgemisches enthaltend eine oder mehrere der Verbindungen der allgemeinen Formel (VII) 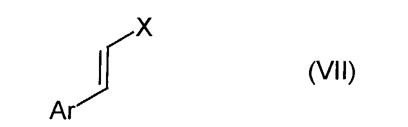 wobei
Ar die für die allgemeine Formel (I) genannte Bedeutung besitzt, und
- X für 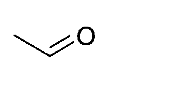 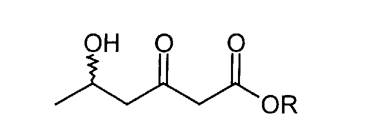 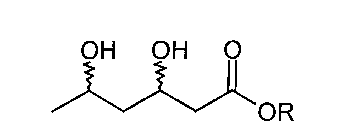 oder 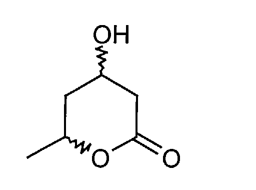 steht, mit Ozon.

10. Verfahren zur Herstellung von Verbindungen der allgemeinen Formel (VIa) 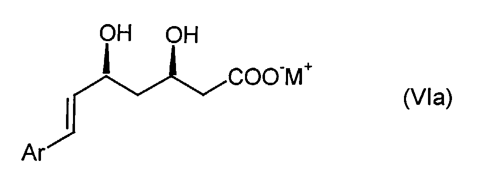 wobei
Ar für einen substituierten oder unsubstituierten Aryl- oder Heteroarylrest steht, und
M für ein Äquivalent eines Alkali- oder Erdalkalimetalls steht,
durch
(1) Umsetzung eines Aldehyds der allgemeinen Formel (I) 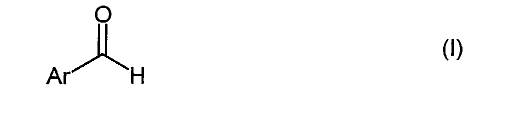 wobei
Ar für einen substituierten oder unsubstituierten Aryl- oder Heteroarylrest steht,
mit einem Phosphonatester oder Triphenylphosphoniumsalz als Kupplungsreagenz in Gegenwart einer organischen oder anorganischen Base unter Bildung einer Verbindung der allgemeinen Formel (II), 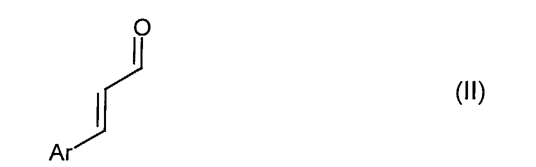 wobei Ar die für die Formel (I) genannte Bedeutung besitzt,
(2) Umsetzung der Verbindung der allgemeinen Formel (II) mit einem Acetessigester eines geradkettigen oder verzweigten C₁-C₆-Alkohols in Gegenwart einer Base unter Bildung einer Verbindung der allgemeinen Formel (III) 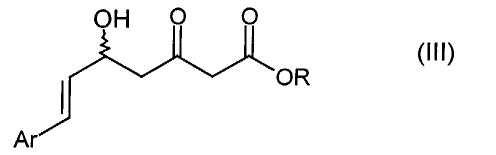 wobei
Ar die für die Formel (I) genannte Bedeutung besitzt und
R für einen geradkettigen oder verzweigten C₁-C₆-Alkylrest steht,
(3) Umsetzung der Verbindung der allgemeinen Formel (III) mit einem Alkylboranreagenz in Gegenwart eines Reduktionsmittels unter Bildung eines Racemates der allgemeinen Formel (IV) 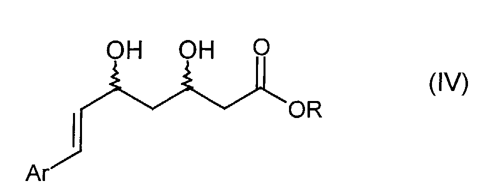 wobei
Ar die für die Formel (I) genannte Bedeutung besitzt und
R die für die Formel (III) genannte Bedeutung besitzt,
(4) Umsetzung des Racemates der allgemeinen Formel (IV) mit einer Base und Cyclisierung unter Bildung eines Racemates der allgemeinen Formel (V) 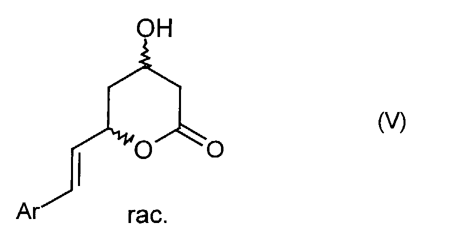 wobei
Ar die für die Formel (I) genannte Bedeutung besitzt,
(5) chromatographische Auftrennung des Racemates der allgemeinen Formel (V) in die enantiomeren Lactone der allgemeinen Formeln (Va) und (Vb) 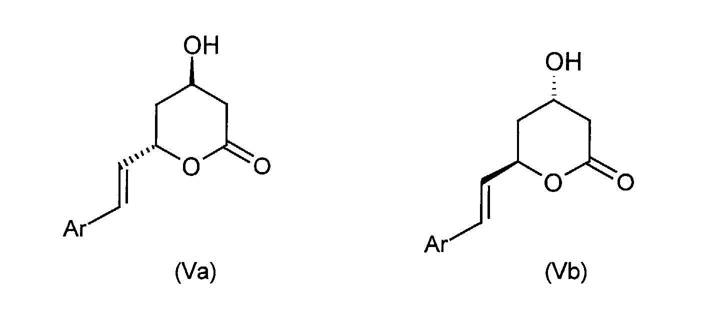 wobei
Ar die für die Formel (I) genannte Bedeutung besitzt,
(6) separate Hydrolyse dieser enantiomeren Lactone der allgemeinen Formeln (Va) und (Vb) unter Bildung der Verbindungen der allgemeinen Formel (VIa) und (VIb) 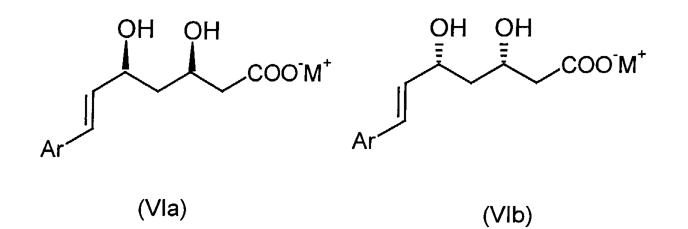 wobei
Ar die für die Formel (I) genannte Bedeutung besitzt, und
M für das Äquivalent eines Alkali- oder Erdalkalimetalls steht.
(7) Umsetzung eines oder mehrerer der Reaktionsgemische, die nach den Schritten (2), (3) und/oder (4) nach Abtrennung der Verbindungen der Formeln (III), (IV) bzw. (V) erhalten werden, enthaltend eine oder mehrere der Verbindungen der allgemeinen Formel (VII) 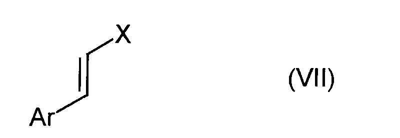 wobei
- X für 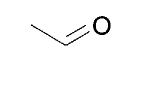 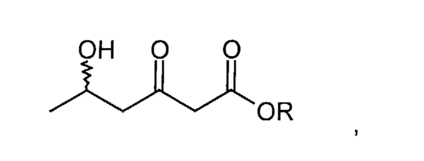 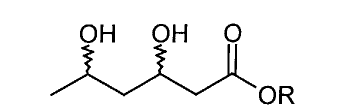 oder 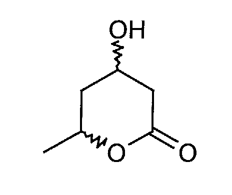 steht,
einzeln oder in vereinigter Form mit Ozon unter Bildung des Aldehyds der allgemeinen Formel (I) und
(8) Rückführung dieses Aldehyds der allgemeinen Formel (I) in Schritt (1).
